# EUROPEAN PATENT APPLICATION

(11) **EP 4 830 706 A1**
(43) Date of publication of application: **28.10.2026**
(21) Application number: 23950882.3
(22) Date of filing: 31.08.2023
(51) Int. Cl.: A61K 31/37, A61P 25/28, A23L 33/10

(54) **COMPOSITION FOR PREVENTING OR TREATING ALZHEIMER'S DISEASE, CONTAINING NOVEL COMPOUND**

(71) Applicant: PRG S&Tech Inc., Busan 46274 (KR)
(72) Inventor: PARK, Bum-Joon, Busan 46241 (KR)
(74) Representative: Mewburn Ellis LLP
(86) International application number: PCT/KR2023/012996
(87) International publication number: WO 2025/048007

(57) **Abstract**

The present invention relates to use of a novel compound for preventing, alleviating, or treating Alzheimer's disease, and the novel compound exhibits an inhibitory effect on tau protein aggregation. In addition, it was identified that presenilin 1 was reduced by treatment using the novel compound. Therefore, the novel compound can be effectively used in the development of a therapeutic agent for Alzheimer's disease.

## Description

### Technical Field

The present disclosure relates to a composition for preventing or treating Alzheimer's disease, including a novel compound.

### Background Art

Alzheimer's disease (AD) is a condition in which memory, behavior, language, and spatial-temporal abilities progressively deteriorate. In the brains of patients with Alzheimer's disease, the formation of plaques caused by amyloid beta is observed, and intracellular neurofibrillary tangles composed of paired helical filaments (PHFs) containing hyperphosphorylated tau are accumulated. As a result, degenerative atrophy occurs throughout brain tissues, inducing the aforementioned symptoms.

In Alzheimer's disease, the deficiency of neurotransmitters such as acetylcholine is a key indicator, and restoring this deficiency has become one of the primary treatment goals.

Parasympathomimetic drugs for Alzheimer's disease can be classified into muscarinic agonists, nicotinic agonists, cholinesterase inhibitors (ChEIs), and drugs that indirectly modulate acetylcholine release. Among these, many ChEIs have been developed and used, including tacrine, physostigmine, donepezil, rivastigmine, and memantine.

Second-generation drugs such as donepezil and rivastigmine have longer durations of action, greater stability, and higher blood-brain barrier (BBB) permeability compared to first-generation drugs like tacrine, leading to higher concentrations within the central nervous system. While first-generation drugs non-selectively inhibit AChE, butyrylcholinesterase, and other peripheral cholinesterases, several newer drugs are known to have higher selectivity for AChE, thereby reducing peripheral side effects. These agents are known to exert their pharmacological action by inhibiting the breakdown of acetylcholine (ACh), thus increasing the concentration of ACh within the synapse.

However, conventional therapeutic agents for Alzheimer's disease are associated with serious side effects upon long-term administration. Therefore, there is a need for the development of new drugs that exhibit equal or superior pharmacological efficacy while reducing such adverse effects.

### Disclosure of the Invention

### Technical Goals

To this end, an object of the present disclosure is to provide a use for preventing, ameliorating, or treating Alzheimer's disease, containing a novel compound.

### Technical Solutions

To achieve the above object, the present disclosure provides a pharmaceutical composition for preventing or treating Alzheimer's disease, including a compound represented by the following Chemical Formula 1 or a pharmaceutically acceptable salt thereof as an active ingredient: 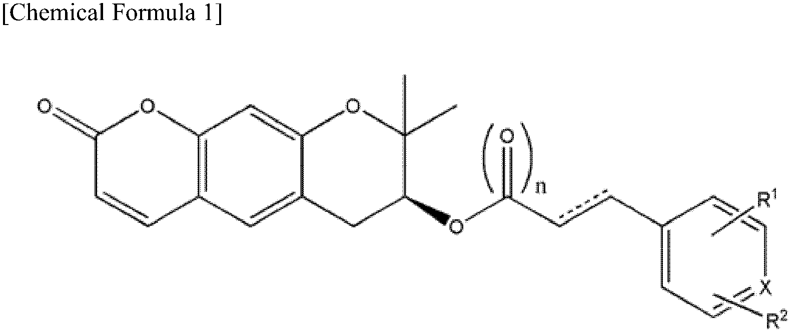
wherein, in the Chemical Formula 1,
- - - - - - is a single bond or a double bond,
n is an integer between 0 and 1,
X is CH or N, and
R¹ and R² may be the same or different and each independently selected from hydrogen, (C1~C4)alkyl, (C1~C4)alkoxy, hydroxy, halo, nitro, cyano, or (C1~C4)alkylcarboxy.

In addition, the present disclosure provides a health functional food composition for preventing or ameliorating Alzheimer's disease, including a compound represented by the Chemical Formula 1 or a pharmaceutically acceptable salt thereof as an active ingredient.

### Advantageous Effects

The present disclosure relates to the use of a novel compound for preventing, alleviating, or treating Alzheimer's disease, wherein the novel compound, PRG-A, exhibited an inhibitory effect on tau protein aggregation. In addition, it was confirmed that treatment with the PRG-A compound led to a reduction in presenilin 1. Consequently, the PRG-A compound can be usefully applied for development as a therapeutic agent for Alzheimer's disease.

### Brief Description of Drawings

Fig. 1 shows the effect of the PRG-A compound in preventing tau tangles. A and B. Tau aggregation induced by overexpression of Tau-WT and Tau-MT was eliminated by compounds PRG-A-01 to PRG-A-04. C. The PRG-A compound alleviated the abnormal tubulin formation caused by overexpression of Tau-MT.
Fig. 2 shows the effect of the PRG-A compound in eliminating presenilin aggregation. A and B. Presenilin-1 (PS1) aggregation induced by overexpression of PS1 was reduced by compounds PRG-A-01 and PRG-A-02. C. The PRG-A compound suppressed PS1 aggregation in a dose-dependent manner.
FIG. 3 shows a cytotoxic effect of a PRG-A compound.

### Best Mode for Carrying Out the Invention

Hereinafter, the present disclosure will be described in detail.

The present disclosure provides a pharmaceutical composition for preventing or treating Alzheimer's disease, including a compound represented by the following Chemical Formula 1, a hydrate thereof, or a salt thereof.

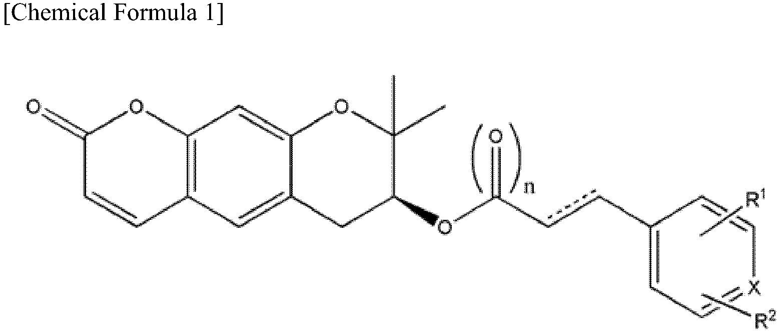

In the Chemical Formula 1,
- - - - - - is a single bond or a double bond,
n is an integer between 0 and 1,
X is CH or N, and
R¹ and R² may be the same or different and each independently selected from hydrogen, (C1~C4)alkyl, (C1~C4)alkoxy, hydroxy, halo, nitro, cyano, or (C1~C4)alkylcarboxy.

Preferably, in the compound, when - - - - - - is a single bond, n is 1, X is CH, and R¹ and R² may be the same or different and each independently selected from (C1~C4)alkoxy, hydroxy, or (C1~C4)alkylcarboxy, but are not limited thereto.

Preferably, in the compound, when - - - - - - is a double bond, n is an integer between 0 and 1, X is CH or N, and R¹ and R² may be the same or different and each independently selected from hydrogen, (C1~C4)alkoxy, hydroxy, halo, nitro, or (C1~C4)alkylcarboxy, but are not limited thereto.

Preferably, the compound may be a compound represented by the following Chemical Formula 2.

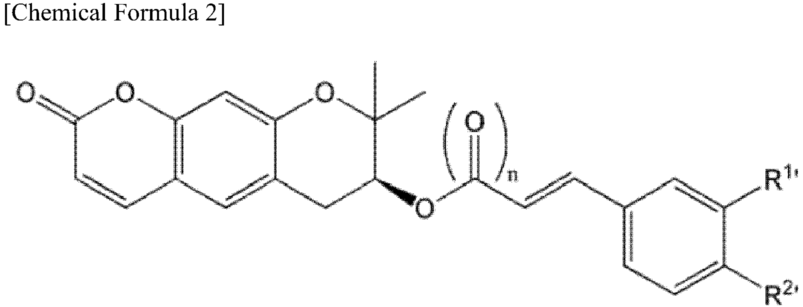

In the Chemical Formula 2,
n is an integer between 0 and 1, and
R¹' and R²' may be the same or different and each independently selected from hydrogen, (C1~C4)alkyl, (C1~C4)alkoxy, hydroxy, halo, nitro, cyano, or (C1~C4)alkylcarboxy.

The compound represented by the Chemical Formula 1 or Chemical Formula 2 may be named a PRG-A compound.

More preferably, the compound may be selected from the group consisting of, but is not limited to, (S)-8,8-dimethyl-2-oxo-7,8-dihydro-2H,6H-pyrano[3,2-g]chromen-7-yl (E)-3-(4-hydroxy-3-methoxyphenyl)acrylate (PRG-A-01), (S,E)-7-((3-(4-hydroxy-3-methoxyphenyl)allyl)oxy)-8,8-dimethyl-7,8-dihydro-2H,6H-pyrano[3,2-g]chromen-2-one (PRG-A-02), (S)-8,8-dimethyl-2-oxo-7,8-dihydro-2H,6H-pyrano[3,2-g]chromen-7-yl 3-(4-hydroxy-3-methoxyphenyl)propanoate (SNU-C4), (S)-8,8-dimethyl-2-oxo-7,8-dihydro-2H,6H-pyrano[3,2-g]chromen-7-yl (E)-3-(3,4-dimethoxyphenyl)acrylate (SNU-C5), (S)-8,8-dimethyl-2-oxo-7,8-dihydro-2H,6H-pyrano[3,2-g]chromen-7-yl 3-(3,4-dimethoxyphenyl)propanoate (SNU-C7), (S)-8,8-dimethyl-2-oxo-7,8-dihydro-2H,6H-pyrano[3,2-g]chromen-7-yl (E)-3-(pyridin-4-yl)acrylate (SNU-C9), (S)-8,8-dimethyl-2-oxo-7,8-dihydro-2H,6H-pyrano[3,2-g]chromen-7-yl (E)-3-(3-hydroxyphenyl)acrylate (SNU-C10), (S)-8,8-dimethyl-2-oxo-7,8-dihydro-2H,6H-pyrano[3,2-g]chromen-7-yl (E)-3-(4-fluorophenyl)acrylate (PRG-A-03), (S,E)-7-((3-(4-fluorophenyl)allyl)oxy)-8,8-dimethyl-7,8-dihydro-2H,6H-pyrano[3,2-g]chromen-2-one (SNU-C13), (S)-8,8-dimethyl-2-oxo-7,8-dihydro-2H,6H-pyrano[3,2-g]chromen-7-yl (E)-3-(3-acetoxyphenyl)acrylate (SNU-C14), (S)-8,8-dimethyl-2-oxo-7,8-dihydro-2H,6H-pyrano[3,2-g]chromen-7-yl 3-(4-acetoxy-3-methoxyphenyl)propanoate (SNU-C15), (S)-8,8-dimethyl-2-oxo-7,8-dihydro-2H,6H-pyrano[3,2-g]chromen-7-yl (E)-3-(4-acetoxy-3-methoxyphenyl)acrylate (SNU-C17), (S)-8,8-dimethyl-2-oxo-7,8-dihydro-2H,6H-pyrano[3,2-g]chromen-7-yl (E)-3-(3,4-difluorophenyl)acrylate (SNU-C18), and (S,E)-7-((3-(3-methoxy-4-nitrophenyl)allyl)oxy)-8,8-dimethyl-7,8-dihydro-2H,6H-pyrano[3,2-g]chromen-2-one (PRG-A-04).

More preferably, the pharmaceutical composition may inhibit aggregation of tau protein and presenilin 1.

The pharmaceutical composition of the present disclosure may be prepared using pharmaceutically suitable and physiologically acceptable auxiliary agents in addition to the active ingredient, and the auxiliary agents may include solubilizers such as excipients, disintegrants, sweeteners, binders, coating agents, swelling agents, lubricants, glidants, or flavoring agents. The pharmaceutical composition of the present disclosure may be preferably formulated as a pharmaceutical composition by including one or more types of pharmaceutically acceptable carriers in addition to the active ingredient for administration. In a composition formulated as a liquid solution, acceptable pharmaceutical carriers may be those that are sterile and biocompatible and used by mixing saline solution, sterile water, Ringer's solution, buffered saline, albumin injection solution, dextrose solution, maltodextrin solution, glycerol, ethanol, and mixtures of one or more of these components, and other conventional additives, such as antioxidants, buffers, and bacteriostatic agents, may be added as needed. In addition, diluents, dispersants, surfactants, binders, and lubricants may be additionally added to formulate as injectable formulations such as aqueous solutions, suspensions, and emulsions, pills, capsules, granules, or tablets.

The form of the pharmaceutical formulation of the pharmaceutical composition of the present disclosure may be granules, powders, coated tablets, tablets, capsules, suppositories, syrups, juices, suspensions, emulsions, drops or injectable liquids, and sustained-release formulations of active compounds. The pharmaceutical composition of the present disclosure may be administered in a conventional manner via intravenous, intraarterial, intraperitoneal, intramuscular, intraarterial, intraperitoneal, intrasternal, transdermal, intranasal, inhalation, topical, rectal, oral, intraocular, or intradermal routes. The effective amount of the active ingredient of the pharmaceutical composition of the present disclosure refers to an amount required for the prevention or treatment of a disease. Therefore, it may be adjusted according to various factors including the type of disease, severity of the disease, type and content of the active ingredient and other ingredients contained in the composition, type of formulation, and the patient's age, weight, general health status, sex and diet, time of administration, route of administration and secretion rate of the composition, treatment period, and concurrently used drugs. Although not limited to, in the case of adults, for example, when administered once to several times a day, the composition of the present disclosure may be administered at a dosage of 0.01 ng/kg to 10 g/kg when administered once to several times a day.

In addition, the present disclosure provides a health functional food composition for preventing or ameliorating Alzheimer's disease, including a compound represented by the following Chemical Formula 1, a hydrate thereof, or a salt thereof.

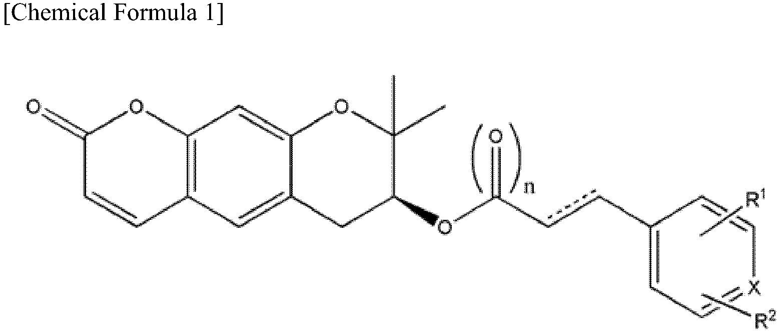

In the Chemical Formula 1,
- - - - - - is a single bond or a double bond,
n is an integer between 0 and 1,
X is CH or N, and
R¹ and R² may be the same or different and each independently selected from hydrogen, (C1~C4)alkyl, (C1~C4)alkoxy, hydroxy, halo, nitro, cyano, or (C1~C4)alkylcarboxy.

Preferably, in the compound, when - - - - - - is a single bond, n is 1, X is CH, and R¹ and R² may be the same or different and each independently selected from (C1~C4)alkoxy, hydroxy, or (C1~C4)alkylcarboxy, but are not limited thereto.

Preferably, in the compound, when - - - - - - is a double bond, n is an integer between 0 and 1, X is CH or N, and R¹ and R² may be the same or different and each independently selected from hydrogen, (C1~C4)alkoxy, hydroxy, halo, nitro, or (C1~C4)alkylcarboxy, but are not limited thereto.

Preferably, the compound may be a compound represented by the following Chemical Formula 2.

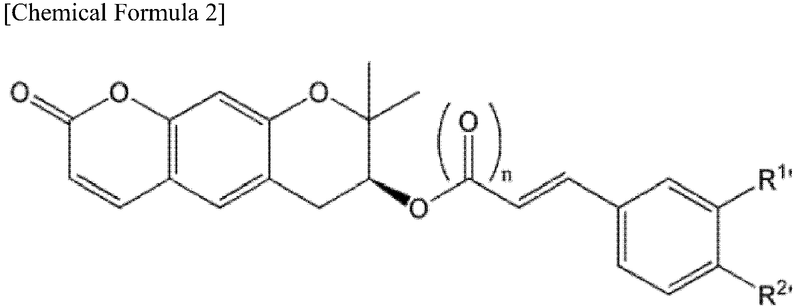

In the Chemical Formula 2,
n is an integer between 0 and 1, and
R¹' and R²' may be the same or different and each independently selected from hydrogen, (C1~C4)alkyl, (Cl~C4)alkoxy, hydroxy, halo, nitro, cyano, or (C1~C4)alkylcarboxy.

The compound represented by the Chemical Formula 1 or Chemical Formula 2 may be named a PRG-A compound.

More preferably, the compound may be selected from the group consisting of, but is not limited to, (S)-8,8-dimethyl-2-oxo-7,8-dihydro-2H,6H-pyrano[3,2-g]chromen-7-yl (E)-3-(4-hydroxy-3-methoxyphenyl)acrylate (PRG-A-01), (S,E)-7-((3-(4-hydroxy-3-methoxyphenyl)allyl)oxy)-8,8-dimethyl-7,8-dihydro-2H,6H-pyrano[3,2-g]chromen-2-one (PRG-A-02), (S)-8,8-dimethyl-2-oxo-7,8-dihydro-2H,6H-pyrano[3,2-g]chromen-7-yl 3-(4-hydroxy-3-methoxyphenyl)propanoate (SNU-C4), (S)-8,8-dimethyl-2-oxo-7,8-dihydro-2H,6H-pyrano[3,2-g]chromen-7-yl (E)-3-(3,4-dimethoxyphenyl)acrylate (SNU-C5), (S)-8,8-dimethyl-2-oxo-7,8-dihydro-2H,6H-pyrano[3,2-g]chromen-7-yl 3-(3,4-dimethoxyphenyl)propanoate (SNU-C7), (S)-8,8-dimethyl-2-oxo-7,8-dihydro-2H,6H-pyrano[3,2-g]chromen-7-yl (E)-3-(pyridin-4-yl)acrylate (SNU-C9), (S)-8,8-dimethyl-2-oxo-7,8-dihydro-2H,6H-pyrano[3,2-g]chromen-7-yl (E)-3-(3-hydroxyphenyl)acrylate (SNU-C10), (S)-8,8-dimethyl-2-oxo-7,8-dihydro-2H,6H-pyrano[3,2-g]chromen-7-yl (E)-3-(4-fluorophenyl)acrylate (PRG-A-03), (S,E)-7-((3-(4-fluorophenyl)allyl)oxy)-8,8-dimethyl-7,8-dihydro-2H,6H-pyrano[3,2-g]chromen-2-one (SNU-C13), (S)-8,8-dimethyl-2-oxo-7,8-dihydro-2H,6H-pyrano[3,2-g]chromen-7-yl (E)-3-(3-acetoxyphenyl)acrylate (SNU-C14), (S)-8,8-dimethyl-2-oxo-7,8-dihydro-2H,6H-pyrano[3,2-g]chromen-7-yl 3-(4-acetoxy-3-methoxyphenyl)propanoate (SNU-C15), (S)-8,8-dimethyl-2-oxo-7,8-dihydro-2H,6H-pyrano[3,2-g]chromen-7-yl (E)-3-(4-acetoxy-3-methoxyphenyl)acrylate (SNU-C17), (S)-8,8-dimethyl-2-oxo-7,8-dihydro-2H,6H-pyrano[3,2-g]chromen-7-yl (E)-3-(3,4-difluorophenyl)acrylate (SNU-C18), and (S,E)-7-((3-(3-methoxy-4-nitrophenyl)allyl)oxy)-8,8-dimethyl-7,8-dihydro-2H,6H-pyrano[3,2-g]chromen-2-one (PRG-A-04).

The health functional food composition of the present disclosure may further include one or more additives selected from the group consisting of organic acid, phosphate, antioxidant, lactose casein, dextrin, glucose, sugar, and sorbitol. The organic acid may be, but is not limited to, citric acid, fumaric acid, adipic acid, lactic acid, or malic acid, the phosphate may be, but is not limited to, sodium phosphate, potassium phosphate, acid pyrophosphate or polyphosphate, and the antioxidant may be, but is not limited to, a natural antioxidant such as polyphenols, catechins, alpha-tocopherol, rosemary extract, licorice extract, chitosan, tannic acid, or phytic acid.

In another specific example of the present disclosure, the health functional food may contain, in addition to the active ingredient, various nutrients, vitamins, minerals (electrolytes), flavoring agents such as synthetic flavoring agents and natural flavoring agents, coloring agents and thickening agents (cheese, chocolate, etc.), pectic acid and salts thereof, alginic acid and salts thereof, organic acids, protective colloid thickeners, pH regulators, stabilizers, preservatives, glycerin, alcohol, and carbonating agents used in carbonated beverages. Additionally, a food composition according to one embodiment of the present disclosure may contain fruit pulp for producing natural fruit juice, fruit juice beverage, and vegetable beverage.

According to one embodiment of the present disclosure, the formulation of the health functional food may be in the form of a solid, powder, granule, tablet, capsule, liquid, or beverage, but is not limited thereto.

In addition, the health functional food may be used in the manufacture of foods such as, but not limited to, confectionery, sugar, ice cream products, dairy products, meat products, fish meat products, tofu or jelly, edible oils, noodles, tea, beverages, special nutritional foods, health supplements, seasoned foods, ice, ginseng products, kimchi pickled foods, dried foods, fruits, vegetables, dried fruits or vegetables, cut products, fruit juice, vegetable juice, mixed juices thereof, chips, noodles, processed livestock foods, processed fishery foods, processed dairy foods, fermented dairy foods, legume foods, grain foods, microbial fermented foods, confectionery and bakery products, seasonings, processed meat products, acidic beverages, licorice, and herbs.

### Modes for Carrying Out the Invention

Hereinafter, in order to help understand the present disclosure, examples will be described in detail. However, the following examples are only intended to illustrate the content of the present disclosure, and the scope of the present disclosure is not limited to the following examples. The examples of the present disclosure are provided to more completely describe the present disclosure to those of ordinary skill in the art.

### <Experimental Example>

The following experimental examples are intended to provide experimental examples that are commonly applied to each embodiment according to the present disclosure.

### 1. Compound

The chemical structures and NMR data of compounds used in the present disclosure are as follows.

### (S)-8,8-dimethyl-2-oxo-7,8-dihydro-2H,6H-pyrano[3,2-g]chromen-7-yl (E)-3-(4-hydroxy-3-methoxyphenyl)acrylate (PRG-A-01)

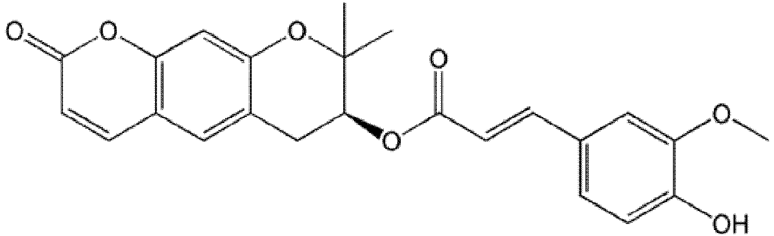

¹H NMR (acetone-d₆, 400MHz)

δ ppm 7.843(d, J=9.6Hz, 1H), 7.616(d, J=16.0Hz, 1H), 7.424(s, 1H), 7.357(s, 1H), 7.123(dd, J=2.0, 8.0Hz, 1H), 6.851(d, J=8.4Hz, 1H), 6.740(s, 1H), 6.387(d, J=15.6Hz, 1H), 6.198(d, J=9.6Hz, 1H), 5.221(t, J=4.6Hz, 1H), 3.895(s, 3H), 3.321(dd, J=4.6, 17.2Hz, 1H), 2.963(dd, J=4.4, 17.6Hz, 1H), 1.421(s, 3H), 1.413(s, 3H); MS(m/z) 423 (M+H)⁺

### (S,E)-7-((3-(4-hydroxy-3-methoxyphenyl)allyl)oxy)-8,8-dimethyl-7,8-dihydro-2H,6H-pyrano[3,2-g]chromen-2-one (PRG-A-02)

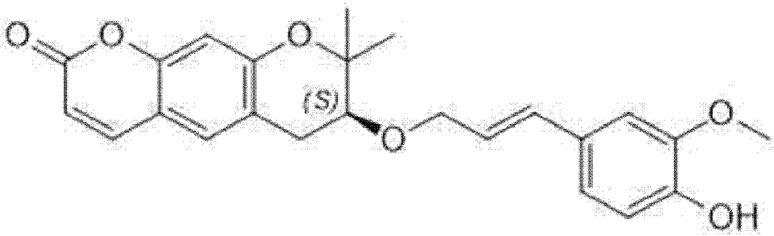

¹H NMR: EW15731-164-P1D4 (400 MHz, CDCl₃)
δ 7.58 (d, J = 12 Hz, 1H), 7.16 (s, 1H), 6.90 - 6.86 (m, 3H), 6.78 (s, 1H), 6.51 (d, J =16 Hz, 1H), 6.22 (d, J = 8.0 Hz, 1H), 6.14 - 6.10 (m, 1H), 5.65 (s, 1H), 4.32 - 4.30 (m, 1H), 4.22- 4.20 (m, 1H), 3.91 (s, 3H), 3.61 - 3.58 (m, 1H), 3.11 - 3.05 (m, 1H), 3.89 - 3.87 (m, 1H), 1.43 (s, 3H), 1.36 (s, 3H).

### (S)-8,8-dimethyl-2-oxo-7,8-dihydro-2H,6H-pyrano[3,2-g]chromen-7-yl (E)-3-(4-fluorophenyl)acrylate (PRG-A-03)

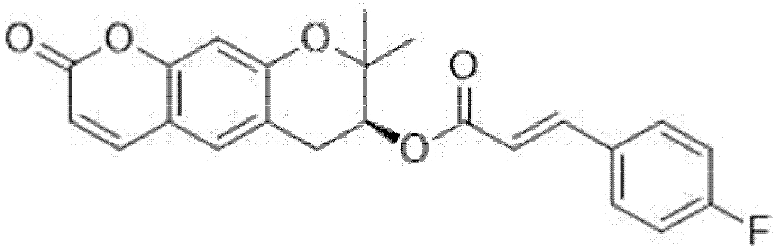

¹H NMR (CD₂Cl₂, 400MHz): δ ppm 7.66 - 7.55 (m, 2H), 7.55 - 7.46 (m, 2H), 7.18 (s, 1H), 7.11 - 7.01 (m, 2H), 6.76 (s, 1H), 6.35 (d, J = 16.0 Hz, 1H), 6.16 (d, J = 9.5 Hz, 1H), 5.17 (app.t, J = 4.7 Hz, 1H), 3.23 (ddd, J = 17.3, 4.8, 1.2 Hz, 1H), 2.92 (dd, J = 17.3, 4.7 Hz, 1H), 1.40 (s, 3H), 1.36 (s, 3H).

### (S,E)-7-((3-(3-methoxy-4-nitrophenyl)allyl)oxy)-8,8-dimethyl-7,8-dihydro-2H,6H-pyrano[3,2-g]chromen-2-one (PRG-A-04)

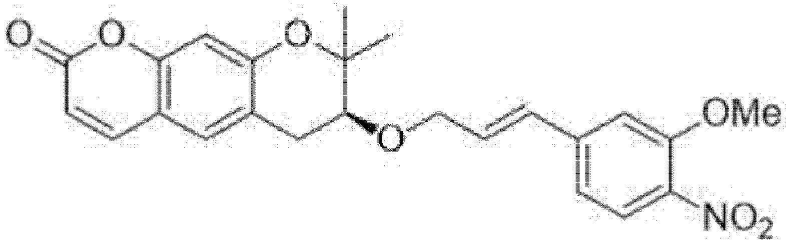

¹H NMR (CDCl₃, 400MHz): δ ppm 7.85 (d, J = 8.4 Hz, 1H), 7.57 (d, J = 9.5 Hz, 1H), 7.16 (s, 1H), 7.05 - 6.97 (m, 2H), 6.78 (s, 1H), 6.60 (app.dt, J = 15.9, 1.7 Hz, 1H), 6.40 (app.dt, J = 15.9, 5.5 Hz, 1H), 6.22 (d, J = 9.4 Hz, 1H), 4.39 (ddd, J = 13.6, 5.4, 1.7 Hz, 1H), 4.24 (ddd, J = 13.4, 5.5, 1.6 Hz, 1H), 3.97 (s, 3H), 3.59 (dd, J = 7.1, 4.9 Hz, 1H), 3.11 (dd, J = 16.7, 4.9 Hz, 1H), 2.87 (dd, J = 16.7, 7.2 Hz, 1H), 1.43 (s, 3H), 1.38 (s, 3H).

### (S,E)-7-((3-(4-fluorophenyl)allyl)oxy)-8,8-dimethyl-7,8-dihydro-2H,6H-pyrano[3,2-g]chromen-2-one (SNU-C13)

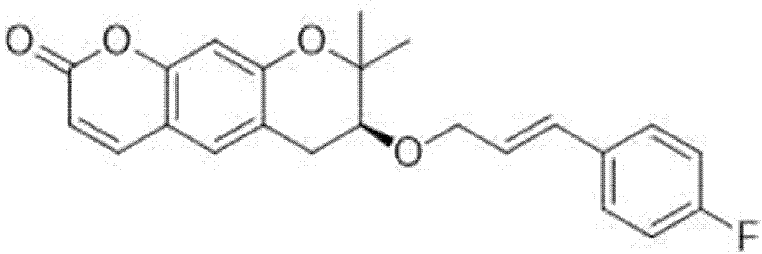

¹H NMR (CDCl₃, 400MHz): δ ppm 7.57 (d, J = 9.5 Hz, 1H), 7.38 - 7.29 (m, 2H), 7.16 (s, 1H), 7.06 - 6.93 (m, 2H), 6.77 (s, 1H), 6.55 (d, J = 15.8 Hz, 1H), 6.25 - 6.13 (m, 2H), 4.33 (ddd, J = 12.8, 5.8, 1.5 Hz, 1H), 4.19 (ddd, J = 12.8, 6.3, 1.4 Hz, 1H), 3.58 (dd, J = 7.4, 5.0 Hz, 1H), 3.08 (dd, J = 16.4, 5.0 Hz, 1H), 2.85 (ddd, J = 16.4, 7.4, 1.1 Hz, 1H), 1.42 (s, 3H), 1.35 (s, 3H).

### (S)-8,8-dimethyl-2-oxo-7,8-dihydro-2H,6H-pyrano[3,2-g]chromen-7-yl (E)-3-(4-acetoxy-3-methoxyphenyl)acrylate (SNU-C17)

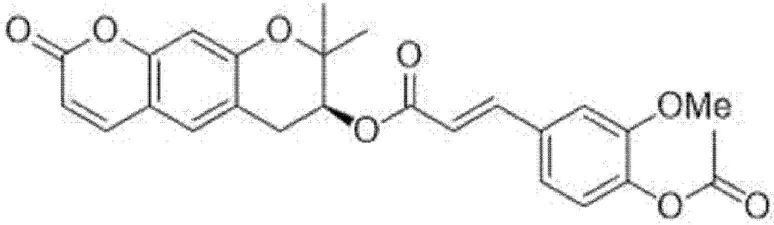

¹H NMR (CD₂Cl₂, 600MHz): δ ppm 7.67 - 7.56 (m, 2H), 7.21 (s, 1H), 7.14 - 7.09 (m, 2H), 7.03 (d, J = 8.0 Hz, 1H), 6.78 (s, 1H), 6.41 (d, J = 16.0 Hz, 1H), 6.19 (d, J = 9.5 Hz, 1H), 5.20 (app.t, J = 4.7 Hz, 1H), 3.82 (s, 3H), 3.26 (ddd, J = 17.3, 4.8, 1.2 Hz, 1H), 2.95 (dd, J = 17.3, 4.5 Hz, 1H), 2.27 (s, 3H), 1.43 (s, 3H), 1.38 (s, 3H).

### (S)-8,8-dimethyl-2-oxo-7,8-dihydro-2H,6H-pyrano[3,2-g]chromen-7-yl (E)-3-(3,4-difluorophenyl)acrylate (SNU-C18)

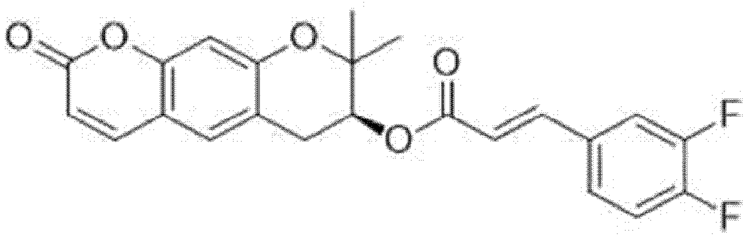

¹H NMR (CDCl₃, 400MHz): δ ppm 7.62 - 7.53 (m, 2H), 7.32 (ddd, J = 11.1, 7.6, 2.2 Hz, 1H), 7.24 - 7.11 (m, 3H), 6.83 (s, 1H), 6.33 (d, J = 16.0, 1H), 6.24 (d, J = 9.4 Hz, 1H), 5.19 (app.t, J = 4.7 Hz, 1H), 3.25 (ddd, J = 17.4, 4.8, 1.1 Hz, 1H), 2.93 (dd, J = 17.4, 4.5 Hz, 1H), 1.43 (s, 3H), 1.39 (s, 3H).

### (7S)-(+)-3-(2-Furanyl)-acrylic acid, 8,8-dimethyl-2-oxo-6,7- dihydro-2H,8H-pyrano[3,2-g]chromen-7-yl-ester (SLC-B050)

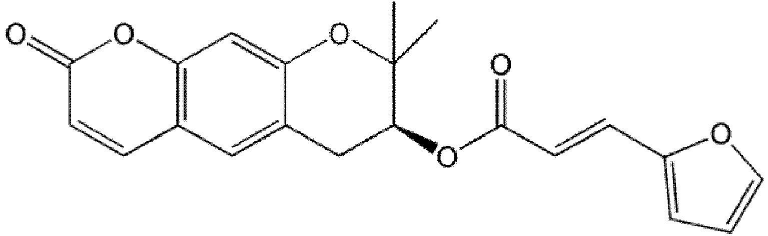

¹H NMR(400 MHz, CDCl₃): δH 7.64(1H, s, H-6'), 7.58(1H, d, J = 9.6Hz, H-4), 7.56(1H, d, J = 16.0Hz, H-3'), 7.41(1H, d, J = 1.6Hz, H-7'), 7.17(1H, s, H-5), 6.82(1H, s, H-10), 6.55(1H, d, J = 1.6Hz, H-8'), 6.23(1H, d, J = 9.6Hz, H-3), 6.13(1H, d, J = 16.0Hz, H-2'), 5.17(1H, t, J = 4.4Hz, H-7), 3.23(1H, dd, J = 4.4, 17.6Hz, H-6a), 2.92(1H, dd, J = 4.4, 17.6Hz, H-6b), 1.42(3H, s, CH3-8), 1.38(3H, s, CH3-8).

### 2. Cell Culture and Reagents

HEK293 cells were obtained from the American Type Culture Collection (ATCC, Manassas, VA, USA) and maintained in DMEM medium supplemented with 10% fetal bovine serum and 1% penicillin-streptomycin at 37°C in a humidified atmosphere with 5% CO₂. SK-N-SH cells were purchased from the Korean Cell Line Bank (KCLB, Seoul, South Korea) and maintained in MEM medium supplemented with 10% fetal bovine serum, 1% antibiotics, 25 mM HEPES, and 300 mg/L L-glutamine. Human fibroblasts (9-year-old female) were obtained from Coriell Cell Repositories (New Jersey, USA) and maintained in EMEM supplemented with 15% FBS, 2 mM glutamine, and 26 mM HEPES, without antibiotics.

### 3. Western blot analysis

For SDS-PAGE, proteins were extracted from cells using RIPA buffer (50 mM Tris-Cl, pH 7.5, 150 mM NaCl, 1% NP-40, 0.1% SDS, and 10% sodium deoxycholate). Samples were separated by SDS-PAGE and transferred to a PVDF membranes. The blotted membrane was blocked with 3% skim milk containing TBST buffer for 1 h and incubated with specific antibodies. Reacted antibodies were detected by ECL and X-ray film exposure. HA (sc-7392) and GFP (green fluorescent protein; sc-8036) antibodies were purchased from Santa Cruz Biotechnology (Santa Cruz, CA, USA). Actin antibody (66009-1-lg) and α-tubulin antibody (66240-1-lg) were obtained from Proteintech (Rosemont, IL, USA). The anti-TH antibody (NB300-109) was purchased from Novus Biologicals (CO, USA). As secondary antibodies, HRP-conjugated goat anti-mouse, goat anti-rabbit, and mouse anti-goat antibodies (Pierce, Thermo Fisher Scientific, Inc., Rockford, IL, USA) were used. After incubation, cells were harvested using TNN buffer (50 mM Tris-Cl, pH 7.5, 150 mM NaCl, 0.3% NP-40) and centrifuged at 14,000 rpm for 30 minutes to separate the pellet (insoluble) and the supernatant (soluble). "Input" refers to whole cell lysates (WCL) harvested using RIPA buffer. Actin was used as a loading control.

### 4. Immunofluorescence staining

Cells on coverslips were washed with PBS, fixed with 4% paraformaldehyde (PFA) for 30 min at room temperature, and then permeabilized in 0.1% Triton X-100/PBS for 10 min. After treating cells with a blocking solution (3% anti-human antibody diluted 1:500 in PBS) for 1 h, cells were incubated with HA (diluted 1:500) overnight at 4°C. Finally, cells were incubated with FITC- and rhodamine-conjugated secondary antibodies for 6 h at 4°C. Nuclei were stained with 4,6-diamidino-2-phenylindole (DAPI), and endoplasmic reticulum (ER) was stained with ER-Tracker Red dye for 10 min. Cells were washed three times with PBS, and then coverslips were mounted with mounting solution (H-5501; Vector Laboratories, Burlingame, CA, USA), followed by analysis with fluorescence microscopy (Zeiss).

### 5. Transfection of vectors

GFP-Tau (WT; #46904, P301L; #46908) plasmids were purchased from Addgene (Cambridge, MA, USA). Transfection was performed using Jet-PEI reagent (JetPEI; Polyplus transfection, New York, NY, USA) according to the manufacturer's protocol. Briefly, the vector was mixed with JetPEI reagent in 150 mM NaCl buffer, and the mixture was incubated for 15 minutes. The mixture was added to cells in serum-free medium for 4 h. After incubation, the cells were replaced with culture medium supplemented with 10% FBS.

### 6. Measurement of cell viability

To investigate cell viability, cells were treated with chemicals (PRG-A compound) for 48 h. Then, the cells were incubated with 0.5 mg/ml 3-(4,5-dimethylthiazol-2-yl)-2,5-diphenyltetrazolium bromide (MTT) solution (475989; Merck, Darmstadt, Germany) at 37°C for 4 h. After removing the excess solution and washing with PBS, the precipitated substances were dissolved in 200 µl of DMSO and quantified by measuring the absorbance at 540 nm.

### <Example 1>

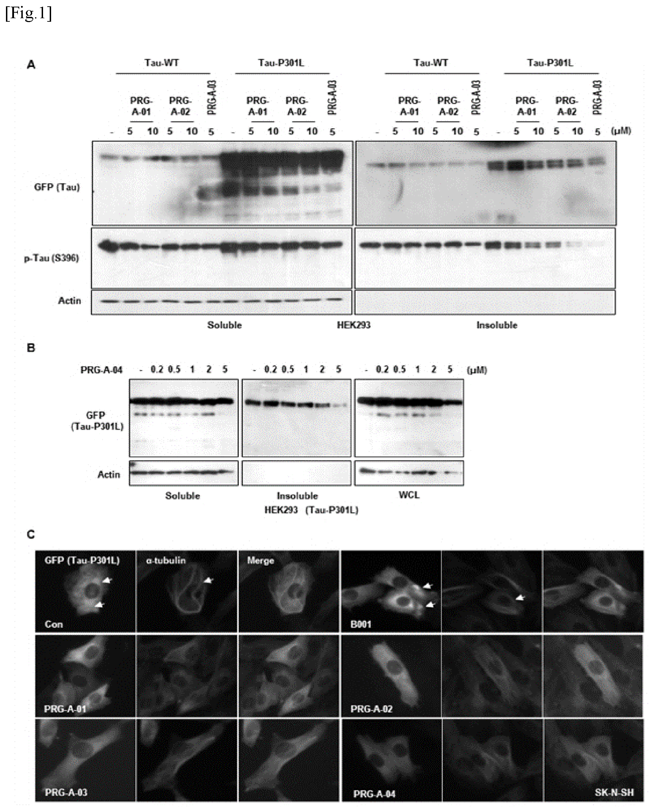

To investigate the effect of the PRG-A compound on tau aggregation (tau tangles), the inventors transfected HEK293 cells with indicator vectors (Tau-WT, Tau-P301L) and treated the cells with selected compounds (5 µM) for 24 hours. Cells were harvested using TNN buffer and centrifuged at 14,000 rpm for 30 minutes to separate the pellet (insoluble) and the supernatant (soluble) (FIGs. 1A and 1B). Aggregated mutant tau (P301L) was recovered as an insoluble fraction upon transfection (FIG. 1A). However, treatment with PRG-A-01 and PRG-A-02 significantly reduced the insoluble mutant tau fraction (FIG. 1A). A similar effect was observed with PRG-A-04 treatment (FIG. 1B). In addition, cells were transfected with the Tau-P301L vector for 24 hours. After incubation, the cells were treated with the PRG-A compound (5 µM) for another 24 hours. Cells were fixed with 4% PFA and immunofluorescence was performed using an α-tubulin antibody (white arrows, strong tau intensity) (FIG. 1C). As a result, abnormal tubulin arrangements (yellow arrows) and mutant tau aggregates (white arrows) were normalized or dissolved by the PRG-A compound (FIG. 1C). The Chem-001 (decursinol) compound showed only marginal effects in inhibiting tau aggregation.

### <Example 2>

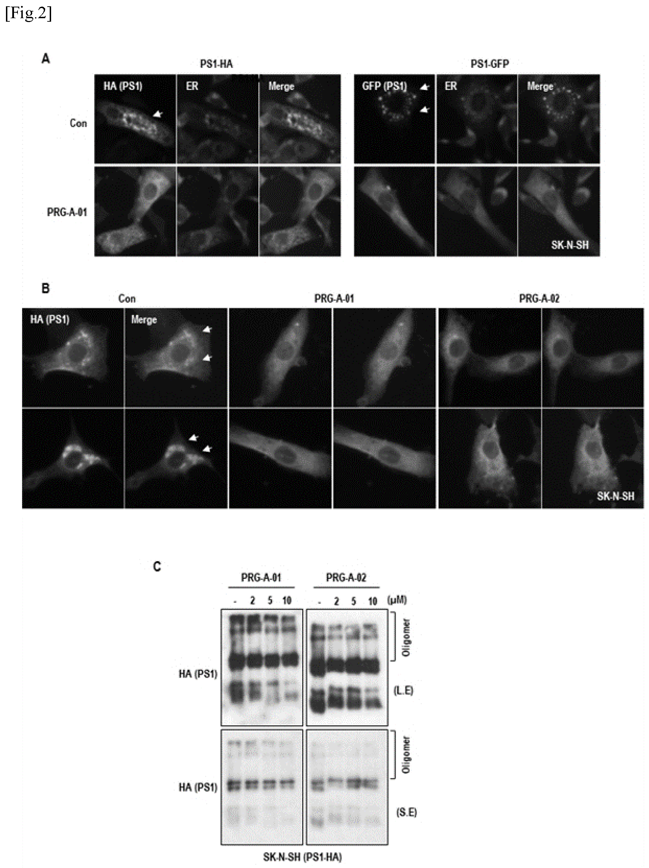

It is also known that mutations in presenilin 1 (PS1) are associated with Alzheimer's disease (AD). Therefore, to examine the effect of the PRG-A compound on PS1 expression and localization, cells were transfected with PS1-HA or PS1-GFP vectors for 24 hours. After incubation, cells were treated with the PRG-A compound (5 µM) for 24 hours. Cells were fixed with 4% PFA and stained with an HA antibody (green) and ER marker (red) to monitor PS1 inclusions (white arrows, strong PS1 intensity) (FIGs. 2A and 2B). Ectopically expressed PS1 formed aggregates in the cytoplasm (FIGs. 2A and 2B). In contrast, the PRG-A compound was able to dissolve PS1 aggregates in IF staining (FIGs. 2A and 2B). To confirm the PS1 aggregation inhibitory effect of the PRG-A compound, SK-N-SH cells were transfected with an indicator vector (PS1-HA), and selected compounds (PRG-A-01 and PRG-A-02) were treated for 24 hours. SDS-PAGE was performed using the HA antibody (FIG. 2C). Indeed, oligomeric PS1 was reduced by treatment with the PRG-A compounds (FIG. 2C). Taken together, these results suggest that the PRG-A compound may also be useful for the prevention or treatment of Alzheimer's disease.

### <Example 3>

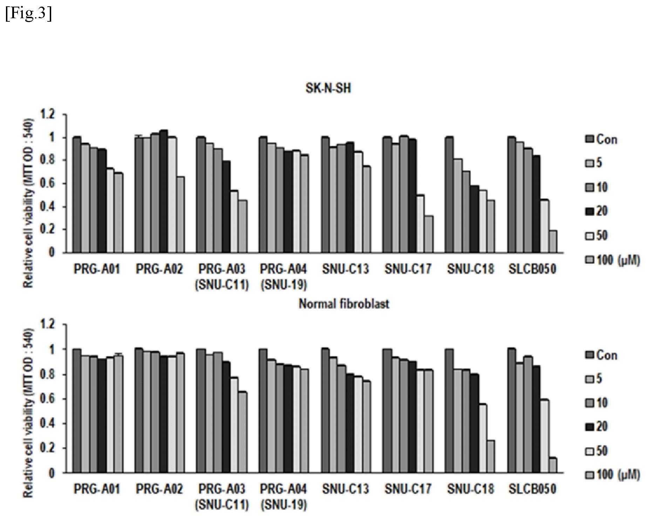

Lastly, to determine the toxicity of PRG-A compounds, normal human fibroblasts and neuroblastoma cells (SK-N-SH) were cultured with each compound for 48 h, and cell viability was measured via the MTT assay. The effect on survival rates of neuronal cells and normal fibroblasts was identified (FIG. 3). The PRG-A-01, PRG-A-02, and PRG-A-04 compounds did not exhibit cytotoxic effects in normal human fibroblasts. The PRG-A-03 compound showed a low impact on cell viability at high doses (50 and 100 µM). In summary, the novel compound did not show toxicity up to 50 µM in normal cells, so the side effects of the new compound are not expected to be serious.

In conclusion, the novel compound may be used to treat Alzheimer's disease (AD) without serious side effects.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. A pharmaceutical composition for preventing or treating Alzheimer's disease, comprising a compound represented by the following Chemical Formula 1, a hydrate thereof, or a salt thereof: 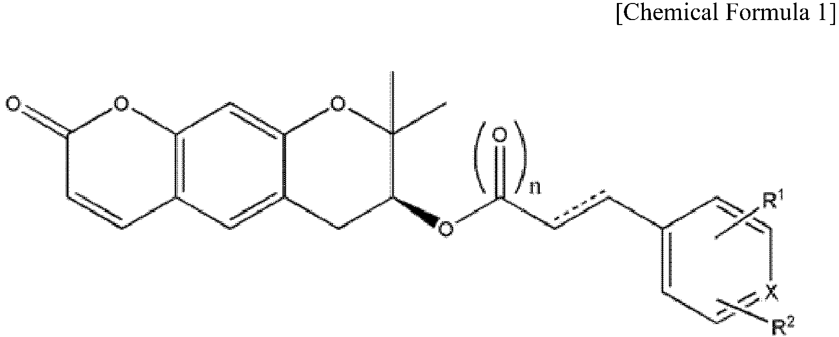
wherein, in the Chemical Formula 1,
- - - - - - is a single bond or a double bond,
n is an integer between 0 and 1,
X is CH or N, and
R¹ and R² are the same or different and each independently selected from hydrogen, (C1~C4)alkyl, (C1~C4)alkoxy, hydroxy, halo, nitro, cyano, or (C1~C4)alkylcarboxy.

2. The pharmaceutical composition of claim 1, wherein, in the compound, when - - - - - - is a single bond, n is 1, X is CH, and R¹ and R² are the same or different and each independently selected from (C1~C4)alkoxy, hydroxy, or (C1~C4)alkylcarboxy.

3. The pharmaceutical composition of claim 1, wherein, in the compound, when - - - - - - is a double bond, n is an integer between 0 and 1, X is CH or N, and R¹ and R² are the same or different and each independently selected from hydrogen, (C1~C4)alkoxy, hydroxy, halo, nitro, or (C1~C4)alkylcarboxy.

4. The pharmaceutical composition of claim 1, wherein the compound is selected from the group consisting of (S)-8,8-dimethyl-2-oxo-7,8-dihydro-2H,6H-pyrano[3,2-g]chromen-7-yl (E)-3-(4-hydroxy-3-methoxyphenyl)acrylate, (S,E)-7-((3-(4-hydroxy-3-methoxyphenyl)allyl)oxy)-8,8-dimethyl-7,8-dihydro-2H,6H-pyrano[3,2-g]chromen-2-one, (S)-8,8-dimethyl-2-oxo-7,8-dihydro-2H,6H-pyrano[3,2-g]chromen-7-yl 3-(4-hydroxy-3-methoxyphenyl)propanoate, (S)-8,8-dimethyl-2-oxo-7,8-dihydro-2H,6H-pyrano[3,2-g]chromen-7-yl (E)-3-(3,4-dimethoxyphenyl)acrylate, (S)-8,8-dimethyl-2-oxo-7,8-dihydro-2H,6H-pyrano[3,2-g]chromen-7-yl 3-(3,4-dimethoxyphenyl)propanoate, (S)-8,8-dimethyl-2-oxo-7,8-dihydro-2H,6H-pyrano[3,2-g]chromen-7-yl (E)-3-(pyridin-4-yl)acrylate, (S)-8,8-dimethyl-2-oxo-7,8-dihydro-2H,6H-pyrano[3,2-g]chromen-7-yl (E)-3-(3-hydroxyphenyl)acrylate, (S)-8,8-dimethyl-2-oxo-7,8-dihydro-2H,6H-pyrano[3,2-g]chromen-7-yl (E)-3-(4-fluorophenyl)acrylate, (S,E)-7-((3-(4-fluorophenyl)allyl)oxy)-8,8-dimethyl-7,8-dihydro-2H,6H-pyrano[3,2-g]chromen-2-one, (S)-8,8-dimethyl-2-oxo-7,8-dihydro-2H,6H-pyrano[3,2-g]chromen-7-yl (E)-3-(3-acetoxyphenyl)acrylate, (S)-8,8-dimethyl-2-oxo-7,8-dihydro-2H,6H-pyrano[3,2-g]chromen-7-yl 3-(4-acetoxy-3-methoxyphenyl)propanoate, (S)-8,8-dimethyl-2-oxo-7,8-dihydro-2H,6H-pyrano[3,2-g]chromen-7-yl (E)-3-(4-acetoxy-3-methoxyphenyl)acrylate, (S)-8,8-dimethyl-2-oxo-7,8-dihydro-2H,6H-pyrano[3,2-g]chromen-7-yl (E)-3-(3,4-difluorophenyl)acrylate, and (S,E)-7-((3-(3-methoxy-4-nitrophenyl)allyl)oxy)-8,8-dimethyl-7,8-dihydro-2H,6H-pyrano[3,2-g]chromen-2-one.

5. The pharmaceutical composition of claim 1, wherein the compound is represented by the following Chemical Formula 2: 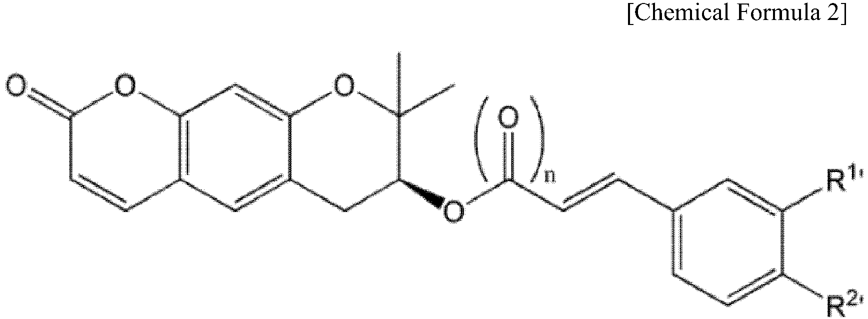
wherein, in the Chemical Formula 2,
n is an integer between 0 and 1, and
R¹' and R²' are the same or different and each independently selected from hydrogen, (C1~C4)alkyl, (C1~C4)alkoxy, hydroxy, halo, nitro, cyano, or (C1~C4)alkylcarboxy.

6. The pharmaceutical composition of claim 1, wherein the pharmaceutical composition inhibits aggregation of tau protein and presenilin 1.

7. A health functional food composition for preventing or ameliorating Alzheimer's disease, comprising a compound represented by the following Chemical Formula 1, a hydrate thereof, or a salt thereof: 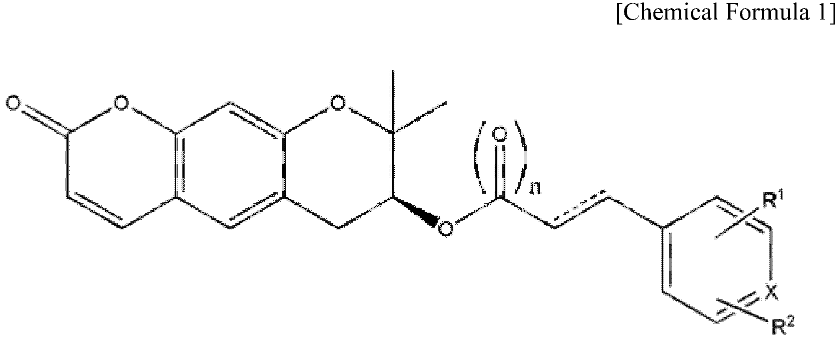
wherein, in the Chemical Formula 1,
- - - - - - is a single bond or a double bond,
n is an integer between 0 and 1,
X is CH or N, and
R¹ and R² are the same or different and each independently selected from hydrogen, (C1~C4)alkyl, (C1~C4)alkoxy, hydroxy, halo, nitro, cyano, or (C1~C4)alkylcarboxy.

8. The health functional food composition of claim 7, wherein, in the compound, when - - - - - - is a single bond, n is 1, X is CH, and R¹ and R² are the same or different and each independently selected from (C1~C4)alkoxy, hydroxy, or (C1~C4)alkylcarboxy.

9. The health functional food composition of claim 7, wherein, in the compound, when - - - - - - is a double bond, n is an integer between 0 and 1, X is CH or N, and R¹ and R² are the same or different and each independently selected from hydrogen, (C1~C4)alkoxy, hydroxy, halo, nitro, or (C1~C4)alkylcarboxy.

10. The health functional food composition of claim 7, wherein the compound is selected from the group consisting of (S)-8,8-dimethyl-2-oxo-7,8-dihydro-2H,6H-pyrano[3,2-g]chromen-7-yl (E)-3-(4-hydroxy-3-methoxyphenyl)acrylate, (S,E)-7-((3-(4-hydroxy-3-methoxyphenyl)allyl)oxy)-8,8-dimethyl-7,8-dihydro-2H,6H-pyrano[3,2-g]chromen-2-one, (S)-8,8-dimethyl-2-oxo-7,8-dihydro-2H,6H-pyrano[3,2-g]chromen-7-yl 3-(4-hydroxy-3-methoxyphenyl)propanoate, (S)-8,8-dimethyl-2-oxo-7,8-dihydro-2H,6H-pyrano[3,2-g]chromen-7-yl (E)-3-(3,4-dimethoxyphenyl)acrylate, (S)-8,8-dimethyl-2-oxo-7,8-dihydro-2H,6H-pyrano[3,2-g]chromen-7-yl 3-(3,4-dimethoxyphenyl)propanoate, (S)-8,8-dimethyl-2-oxo-7,8-dihydro-2H,6H-pyrano[3,2-g]chromen-7-yl (E)-3-(pyridin-4-yl)acrylate, (S)-8,8-dimethyl-2-oxo-7,8-dihydro-2H,6H-pyrano[3,2-g]chromen-7-yl (E)-3-(3-hydroxyphenyl)acrylate, (S)-8,8-dimethyl-2-oxo-7,8-dihydro-2H,6H-pyrano[3,2-g]chromen-7-yl (E)-3-(4-fluorophenyl)acrylate, (S,E)-7-((3-(4-fluorophenyl)allyl)oxy)-8,8-dimethyl-7,8-dihydro-2H,6H-pyrano[3,2-g]chromen-2-one, (S)-8,8-dimethyl-2-oxo-7,8-dihydro-2H,6H-pyrano[3,2-g]chromen-7-yl (E)-3-(3-acetoxyphenyl)acrylate, (S)-8,8-dimethyl-2-oxo-7,8-dihydro-2H,6H-pyrano[3,2-g]chromen-7-yl 3-(4-acetoxy-3-methoxyphenyl)propanoate, (S)-8,8-dimethyl-2-oxo-7,8-dihydro-2H,6H-pyrano[3,2-g]chromen-7-yl (E)-3-(4-acetoxy-3-methoxyphenyl)acrylate, (S)-8,8-dimethyl-2-oxo-7,8-dihydro-2H,6H-pyrano[3,2-g]chromen-7-yl (E)-3-(3,4-difluorophenyl)acrylate, and (S,E)-7-((3-(3-methoxy-4-nitrophenyl)allyl)oxy)-8,8-dimethyl-7,8-dihydro-2H,6H-pyrano[3,2-g]chromen-2-one.

11. The health functional food composition of claim 7, wherein the compound is represented by the following Chemical Formula 2: 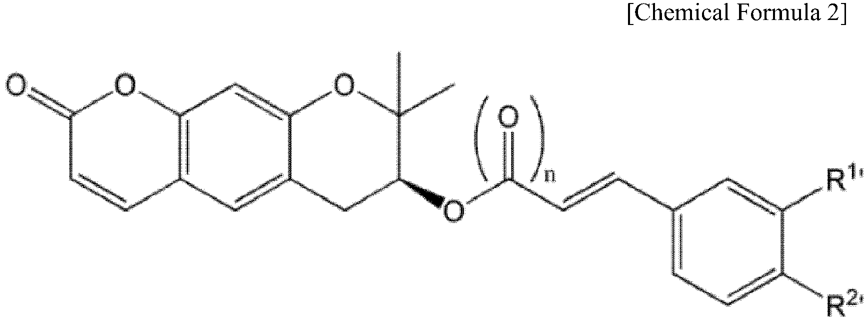
wherein, in the Chemical Formula 2,
n is an integer between 0 and 1, and
R¹' and R²' are the same or different and each independently selected from hydrogen, (C1~C4)alkyl, (C1~C4)alkoxy, hydroxy, halo, nitro, cyano, or (C1~C4)alkylcarboxy.
